# FASCICULE DE BREVET EUROPEEN

(11) **EP 0 453 413 B1**
(45) Date de publication et mention de la délivrance du brevet: **30.11.1994**
(21) Numéro de dépôt: 91830128.4
(22) Date de dépôt: 28.03.1991
(51) Int. Cl.: A61F 5/02, A61F 13/02

(54) **Dispositif de distension pour le traitement des contractures des muscles paravertébraux**
Dehneinrichtung zur Behandlung von Kontrakturen der paravertebralen Muskeln
Distention device for treating contractures of the paravertebral muscles

(30) Priorité: 10.04.1990 IT 936290; 04.10.1990 IT 949990
(43) Date de publication de la demande: 23.10.1991
(73) Titulaire: Giontella, Massimo, I-50125 Firenze (IT)
(72) Inventeur: Ciacca, Giulio, I-06100 Perugia (IT)
(74) Mandataire: Martini, Lazzaro

(56) Documents cités:
- EP-A- 0 247 012
- FR-A- 928 389
- GB-A- 2 215 607
- US-A- 3 068 860
- US-A- 3 528 426

## Description

La présente invention a pour objet un dispositif de distension pour le traitement des contractures des muscles paravertébraux.

Il est connu que les muscles paravertébraux ont pour fonction de soutenir la colonne vertébrale et de permettre son inflexion, son extension et les autres mouvements. Dans certains tableaux pathologiques qui concernent les structures rachidiennes comme les discopathies, la laxité des articulations intervertébrales, les inflammations ligamenteuses et autres, on arrive assez souvent à un blocage des structures rachidiennes avec la contracture antalgique des muscles paravertébraux, qui engendre une scoliose antalgique de sorte que le rachis se courbe de manière permanente sur un côté et que le patient n'est plus en mesure de se redresser.
Et la contracture antalgique peut provoquer une douleur qui peut être supérieure à celle due à la cause qui l'a engendré et provoque, avec celle-ci, un cercle vicieux d'aggravation.

Il est bien connu qu'en effectuant une distension de la musculature contracturée, on peut, dans de nombreux cas, résoudre la symptomatologie clinique et, dans d' autres cas, permettre l'exécution de traitements physiothérapiques spécifiques ou la manipulation vertébrale ou l'application du corset, interventions qui sont difficilement supportables pendant la phase aigue.

On connaît d'après le document FR-A-928.389 un dispositif pour la pratique thérapeutique consistant en une bande de matière élastique qui comporte des fentes longitudinales et une face adhésive à l'épiderme.
Ce dispositif résulte bien capable d'exercer une contraction ou une traction sur des surfaces d'épiderme; par exemple en chirurgie pour maintenir une coupure à l'état de constriction ou, au contraire, une plaie à l'état ouvert ainsi que, dans le demaine de l'esthétique, pour réduire les rides de l'épiderme en combinaison de l'application sur la peau d'un produit astringent. Mais ce dispositif connu, pour le fait qu'il est réalisé en matière élastique et muni d'adhésif seulement à ses deux extrémités, ne permet pas - en aucune façon - de tirer énergiquement pour une période pouvant aller jusqu'à quelques jours les muscles paravertébraux dans la direction opposée à celle de la contracture ni d'obtenire une traction seqmentaire ni une traction de part et d'autre des apophyses épineuses, séparément.

La présente invention a pour but de proposer un dispositif de distension des contractures musculaires paravertébrales, particulièrement adapté pour le traitement de la phase aigue de tableaux de lombalgie et sciatalgie.

Ce résultat a été atteint conformément à l'invention en réalisant un dispositif de distension ayant les caractéristiques prévues dans la revendication 1.

Avantageusement, la face adhésive des bandes, qui est destinée à adhérer sur la peau du patient, est protégée par une feuille de papier à base de silicone qui doit être retirée avant l'application du dispositif de distension.

Les avantages obtenus grâce à la présente invention consistent essentiellement dans le fait qu'il est possible d'obtenir un délassement de la musculature paravertébrale avec une amélioration immédiate de la symptomatologie douloureuse; qu'un dispositif de distension selon l'invention est de réalisation facile, d'application simple et rapide même par le personnel paramédical; qu'il est pratique à porter, facilement tolérable par des patients de n'importe quels âge et constitution physique, et ne présente, en plus, aucune contre-indication.

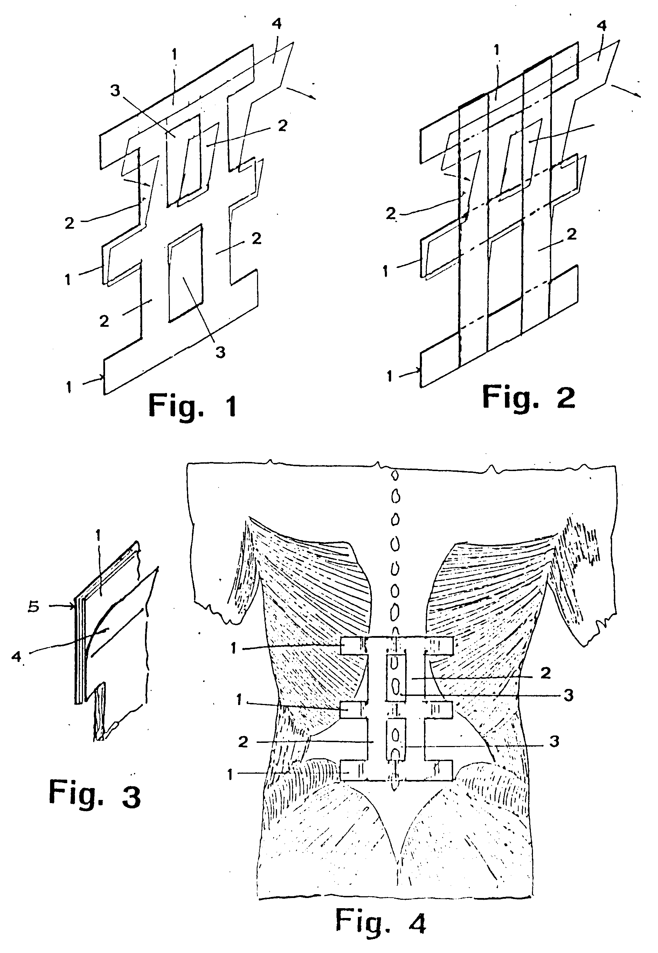

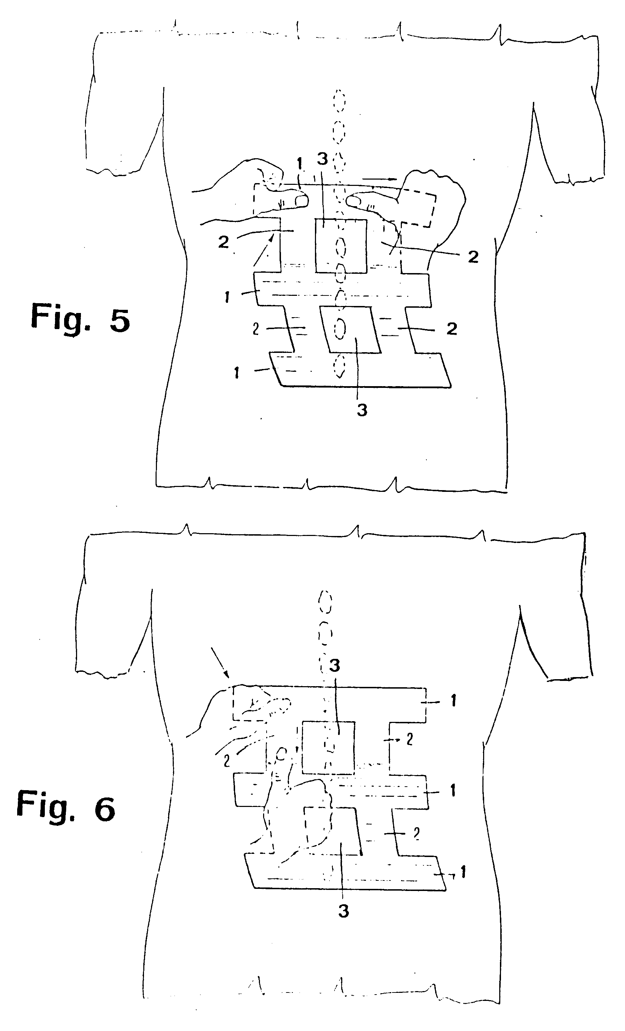

Ces avantages et caractéristiques de l'invention ainsi que d'autres seront plus et mieux compris de chaque homme du métier à la lumière de la description qui va suivre et à l'aide des dessins annexés donnés à titre d'exemplification pratique de l'invention, mais à ne pas considérer dans le sens limitatif; dessins sur lesquels la FIG. 1 représente la vue axonométrique d'ensemble d'un dispositif de distension musculaire selon une première forme de réalisation de l'invention, avant son utilisation; la FIG. 2 représente un dispositif de distension selon une autre forme de réalisation de l'invention; la FIG. 3 représente une vue détaillée du dispositif de distension de la Fig. 1; la FIG. 4 représente le dispositif de distension de la Fig. 1 après son application sur la zone du muscle érecteur de la colonne vertébrale; la FIG. 5 illustre le début de la procédure d'application du dispositif de distension sur le dos du patient; la FIG. 6 illustre la suite de la procédure pour l'application du dispositif de distension.

Réduit à sa structure essentielle et en référence aux Fig. 1 et 3 des dessins annexés, un dispositif de distension pour le traitement de la contracture des muscles paravertébraux conformément à l'invention est constitué d'une plaque avec trois premières bandes 1 parallèles horizontales de ruban adhésif, de largeur suffisante (par exemple 40-50 mm) et d'une longueur d'environ 20-25 cm, qui sont reliées entre elles deux à deux, mais en formant un morceau unique, au moyen de deux bandes parallèles verticales 2 du même ruban adhésif, de même largeur que les premières bandes et d'une longueur d'envrion 4-6 cm de manière à obtenir entre chaque paire de bandes horizontales 1 un évidement médian 3 dépourvu de tissu adhésif d'environ 2-3 cm x 5-6 cm et que les bandes horizontales 1 présentent les deux extrémités qui dépassent de part et d'autre par rapport aux deux bandes verticales 2 d'environ 3-5 cm.
Toutes les bandes 1,2 sont obtenues dans un seul morceau par découpage avec emporte-pièce, dans une pièce de tissu anélastique avec une face auto-adhésive, tel que celui utilisé pour les sparadraps.

Afin d'accroître la robustesse du dispositif de distension, il est prévu, conformément à l'invention, d'utiliser plusieurs tissus anélastiques, auto-adhésifs superposés puis découpés à l'emporte-pièce.

La coplanarité entre les bandes horizontales 1 et celles verticales 2 du dispositif de distension rend l'application manuelle très simple et la fixation sur le dos du patient très efficace.

Pour augmenter la rigidité du dispositif de distension, il est prévu, conformément à l'invention, de munir la face dorsale, c'est-à-dire non adhésive du dispositif, d'une plaque 5 en matière plastique ou en feutre adhésif qui est fixée superposée à la face postérieure du morceau de tissu, avant son découpage par emporte-pièce.

Pour ce qui concerne la feuille 4 de papier à base de silicone, qui protège la face adhésive des bandes 1,2 et à enlever au moment de l'application du dispositif de distension, cette feuille est constituée de préférence de deux sections longitudinales ou transversales, et ceci est d'autant plus avantageux que la longueur des bandes 1,2 est plus grande.

Dans certains cas particuliers, il peut s'avérer utile, dans le but de permettre une action relaxante verticale plus efficace, que les deux bandes verticales 2 soient reliées entre elles par deux bandes horizontales 1 et uniquement en correspondance des extrémités, supérieure et inférieure, de manière à laisser une seule zone médiane 3 privée de tissu adhésif.

En référence à la Fig. 2 des dessins annexés, les bandes horizontales 1 sont superposées à celles verticales 2 de part et d'autre de la face adhésive de ces dernières et unies par couture.

Pour appliquer le dispositif de distension, procéder de la manière suivante.
Après avoir détaché le segment de papier à base de silicone 4 correspondant à la bande adhésive horizontale 1 supérieure, on fixe ladite bande sur le dos du patient en correspondance de la zone de passage dorso-lombaire, avec une manoeuvre de pression transversale à effectuer avec un pouce ou avec les deux pouces (voir Fig. 5); ensuite, après avoir libéré la partie supérieure d'une des bandes adhésives verticales 2 du papier à base de silicone correspondant 4, on applique ladite partie de bande 2 avec une opération combinée de pression et de traction vers le bas à effectuer avec le pouce de la main droite, alors qu'on appuie avec la main gauche sur la bande horizontale 1 supérieure, appliquée précédemment (voir Fig. 6); successivement, de la même manière, on applique la partie correspondante de l'autre bande verticale 2; après quoi, on accroche la bande adhésive horizontale 1 médiane; ensuite, on applique la partie inférieure de chacune des bandes adhésives verticales 2 suivant la même technique de compression et de traction vers le bas; et en dernier, on applique la bande adhésive horizontale 1 inférieure.

Avec ledit dispositif de distension musculaire ainsi appliqué, on a comme résultat que les muscles paravertébraux contracturés, qui se trouvent en dessous des deux bandes adhésives verticales 2, sont énergiquement tirés dans la direction opposée à celle de la contracture, c'est-à-dire vers le bas, pour une durée variable entre quelques heures et quelques jours et donc pour une période de temps suffisante pour leur décontraction.

Il va de soi que les bandes adhésives verticales 2 peuvent être plus de deux, c'est-à-dire quatre ou six, mais dans tous les cas, les deux bandes qui se trouvent au centre doivent être prévues à une distance telle qu'elle permette leur application de chaque côté des apophyses épineuses.

## Revendications

1. Dispositif de distension pour le traitement des contractures des muscules paravertébraux comprenant une plaque modelée de manière à former des bandes horizontales (1) et verticales (2) avec des évidements (3) verticaux et dont une face est adhésive à l'épiderme caractérisé en ce que lesdites bandes (1,2) sont en tissu résistant, anélastique et en ce que lesdits évidement (3) sont alignés selon l'axe médian de la plaque.

2. Dispositif selon la revendication 1 caractérisé en ce que lesdits évidements (3) verticaux de la plaque ont une largeur permettant de loger les apophyses épineuses du patient.

## Claims

1. Distention device for the treatment of vertebral muscle contractions, consisting of a plate made to form horizontal (1) and vertical (2) strips, with vertical hollows (3) and where the surface adheres to the skin, characterized in that the said strips ( 1,2 ) are of resistant inelastic fabric and that the said hollows (3) are aligned along the medial axis of the plate.

2. Device according to Claim 1, characterized in that the said vertical hollows (3) of the plate have a width enabling them to accommodate the spinal apophyses ( projections ) of the patient.

## Patentansprüche

1. Lockerungsvorrichtung für die Behandlung von Verspannungen der neben der Wirbelsäule liegenden Muskeln, die eine Platte umfaßt, welche in der Weise ausgebildet ist, daß sie horizontale (1) und vertikale (2) Bänder mit vertikalen Ausnehmungen (3) umfaßt, und deren eine Fläche an der Haut anhaftet, dadurch **gekennzeichnet**, daß die Bänder (1, 2) aus einem widerstandsfähigen, unelastischen Gewebe bestehen und daß die Ausnehmungen (3) längs der Mittelachse der Platte ausgerichtet sind.

2. Vorrichtung nach Anspruch 1, dadurch **gekennzeichnet,** daß die vertikalen Ausnehmungen der Platte eine Größe besitzen, die es ermöglicht, in ihnen die Dornfortsätze des Patienten unterzubringen.
